# EUROPEAN PATENT APPLICATION

(11) **EP 4 169 507 A1**
(43) Date of publication of application: **26.04.2023**
(21) Application number: 21203972.1
(22) Date of filing: 21.10.2021
(51) Int. Cl.: A61K 9/14

(54) **COMPOSITION, METHOD FOR ITS PREPARATION AND ITS USE FOR INCREASING THE SOLUBILITY OF ACTIVE SUBSTANCES**

(71) Applicant: PHILIPPS-UNIVERSITÄT MARBURG, 35037 Marburg (DE)
(72) Inventor: Keck, Cornelia, 14548 Schwielowsee (DE)
(74) Representative: Durm Patentanwälte PartG mbB

(57) **Abstract**

The present application relates to a composition for increasing the solubility of an active substance, comprising crushed eggshell, on or in which the active substance is sorbed. Furthermore, a method for producing the composition as well as its use are described

## Description

The present invention relates to a composition, a method for its preparation and its use for increasing the solubility of active substances.

Active substances can only be absorbed by the body if they are available dissolved at the site of absorption. In many cases, this is a problem today, i.e., many substances cannot be absorbed by the body, or only inadequately, due to poor solubility. In pharmacy, this affects up to 90% of all newly synthesized active ingredients. Strategies must therefore be found to make such active ingredients readily soluble.

To date, there are various strategies to improve solubility, including so-called nanocrystals, solid solutions, mixed crystals, porous materials, such as porous silica, etc.

Nanocrystals are the most commonly used formulation strategies to date regarding the number of products on the market. Disadvantage here is that the solubility can only be increased by a factor of about 2-3.

Mixed crystals and solid solutions achieve good increases in solubility. However, these are not universal strategies, as individual formulations must be developed for each individual active ingredient. This is time-consuming and cost-intensive. The additives used for this purpose are often expensive and less environmentally friendly. This applies to production and also to biodegradability. The number of market products is correspondingly small.

Porous materials are comparatively to be regarded as a relatively "young" technology for increasing solubility. The principle recognized to date for increasing solubility is the incorporation of active ingredients into nanoscale pores (mesopores) in amorphous form. The increase of solubility with this method is very good (usually 3-10 times). The disadvantage of the method is the use of silicate particles, which cannot be degraded by the body (and nature) and which - depending on their origin - may also contain higher amounts of aluminium (suspected promotion for the manifestation of Alzheimer's disease). The production of the porous silicate particles is still expensive and not environmentally friendly. Producing tablets of the porous materials is only possible with the addition of various excipients, i.e., the amount of added silicate particles is < 10%, so that the total amount of active ingredient (loaded into the silicate particles) is also correspondingly small.

M. M. Than (Mahidol University Journal of Pharmaceutical Sciences 2012; 39 (3-4), 32-38) describes the utilization of eggshell powder as excipient in fast and sustained release acetaminophen tablets. The active substance, in this case acetaminophen, is only physically mixed with the eggshell powder, but it is not sorbed thereto.

The technical object underlying the present invention is seen in providing particles, a method for its production and its use that are made of harmless and preferably biodegradable materials (both in the body and in the environment), that can be produced in a cost-effective and environmentally friendly manner, from renewable raw materials and that also offer a high loading capacity for the active ingredients in the final formulation.

This object is solved by the independent claims. Preferred embodiments are defined in the dependent claims.

According to the present invention, there is provided a composition for increasing the solubility of an active substance, comprising crushed eggshell, on or in which the active substance is sorbed.

Sorption as used herein is a collective term for processes that lead to an accumulation of a substance within a phase or at an interface between two phases. The enrichment within a phase is more precisely called absorption, that at the interface adsorption. Accordingly, the term "sorbed" as used according to the present invention encompasses absorption, adsorption as well as a mixture of both.

In one embodiment, the composition according to the present invention is obtainable by dissolving the active substance in a solvent, which is different from a solvent in which the solubility of the active substance shall be increased, in order to obtain a solution, mixing the solution with the crushed eggshell and optionally removing at least a part of the solvent, in which the active substance is dissolved. The detailed process steps are explained in more detail in the following when the method according to the present invention is described so that in so far it is referred to the following. It is pointed out that it is not clear whether the process of sorbtion of the active substance to the crushed eggshell is adsorption, absorption or a mixture of both so that an adequate way to describe the composition is the method for its preparation.

Furthermore, the active substance can be hardly soluble or insoluble in one solvent but be readily soluble in another solvent. For carrying out the process for preparing the composition according to the present invention, it goes without saying that a solvent is chosen that dissolves the active substance but this is not the solvent in which the solubility of the active substance shall be increased.

The term "active substance" means any substance which has a desired effect. In particular, active substances are substances that have a specific effect or cause a specific reaction in an organism. In one embodiment, the active substance is an active pharmaceutical ingredient. Usually the solubility of a substance shall be increased which has a poor solubility, in particular the physiological environment of a human or animal body. The active substance as used in the composition according to the present invention may be sparingly soluble, poorly soluble, very poorly soluble or practically insoluble, wherein sparingly soluble means 30 parts to 100 parts, poorly soluble means 100 parts to 1 000 parts, very poorly soluble means 1 000 parts to 10 000 parts and practically insoluble means more than 10 000 parts volume solvent for 1 mass part substance at 15 °C to 25 °C.

The composition according to the present invention contains crushed eggshells. As egg for obtaining the eggshells any egg can be used, but hen's eggs are preferred since they are readily available, and pharmaceutically acceptable because they are used as food product. The term "crushed" indicates that the eggshell has a smaller size compared to the egg itself. Crushed eggshells are commercially available for the nutrition of dogs and cats.

In one embodiment, the crushed eggshell is present as a ground, powdered, micronized or nanonized material. During grinding, the eggshell is crushed (with a grinder) by grating and crushing. A powder as used herein is a (nearly) dust finely crushed, pulverized, ground substance. Micronization is the significant reduction of the average particle size, wherein the usual particle sizes between 100 µm and 1000 µm are reduced to a spectrum of 2 µm to 200 µm. In the nanonized material the crushed eggshell is present in the nanoscale. These are usual techniques for crushing materials. The skilled person knows methods and materials to carry them out. The crushing in the nanoscale can be carried out in the presence of a liquid.

In one embodiment, the crushed eggshell is present as a coarse-grained powder, a medium-fine powder, a fine powder, or a very fine powder. The fineness of powders can be characterized by specifying one or two sieve numbers, determining the percentage m/m of material passing through each sieve. The sieve table of Ph. Eur. lists 18 standardized sieves; as sieve numbers it uses the clear square mesh sizes in µm: 11 200; 8 000; 5 600; 4 000; 2 800; 2 000; 1 400; 1 000; 710; 500; 355; 250; 180; 125; 90; 63; 45; 38. When two sieves are used, the sieve size (A) which is passed by equal or more than 95 % of the samples and a second sieve size (B) which is passed by equal or less than 40 % are determined. Based on this method, Ph. Eur. defines four grain classes:

| | | |
|---|---|---|
| Grain class | Sieve (A) | Sieve (B) |
| coarse-grain | 1400 | 355 |
| medium fine | 355 | 180 |
| fine | 180 | 125 |
| very fine | 125 | 90 |

In one embodiment, the crushed eggshell is loaded with from 0.1% to 100% of the maximum loading capacity of the crushed eggshell with the active substance.

In a further embodiment, the composition is present in suspensions, creams, gels, ointments, pastes, capsules, granules, pellets, tablets, drops, sprays, suppositories, lozenges, patches, pens. These are usual formulations in the field of medicaments. The skilled person knows the methods and the materials for obtaining these formulations, i.e. to provide the composition according to the present invention in these formulations.

In one embodiment, the composition according to the present invention further contains a compound selected from the group consisting of C3-C10 trihydroxy compounds, for example glycerol, and/or an acid, for example citric acid. It has been surprisingly found that the addition of these further compounds increase the passive penetration of the active substance for example the penetration through the skin.

The present invention has shown that the composition according to the present invention can be obtained by simply adding crushed eggshells (ground, powdered, micronized or nanonized) to active ingredient solutions. After removing, for example evaporation, of the solvent, the active ingredient accumulates in or on the eggshell particles, increasing its solubility by up to 5-fold. The increased solubility leads to improved active ingredient penetration. Poorly soluble active ingredients can be made readily bioavailable with the technology. Fields of application are oral application, topical application. For application in or on humans, animals or other surfaces (e.g. for application of poorly soluble fertilizers, pesticides, surface disinfection, etc.).

Eggshells are approved as foodstuffs and therefore harmless in their application. They can be obtained from renewable raw materials and, as a waste product, are particularly readily and inexpensively available. Any type of active substance can be loaded onto and/or into eggshells. It is therefore a universal method. Eggshells loaded with active substances (or unloaded) can either be applied directly as powder or incorporated into liquid or semi-solid formulations, filled into capsules or simply compressed into tablets. Studies show that the tablets obtained in this way meet the regulatory requirements of the European Pharmacopoeia. Eggshell formulations can thus be used as a new, cost-effective and particularly environmentally friendly method for improving the efficacy of poorly soluble substances in a wide range of sectors (pharmaceuticals, healthcare, food, cosmetics, textile industry, paints and coatings, agriculture, etc.). The principle is simple, but smart, i.e. easy to produce, applicable, and therefore has a high utility.

The present invention further relates to a method for preparing a composition for increasing the solubility of an active substance, in particular the above described composition according to the present invention, comprising crushed eggshell, on or in which the active substance is sorbed, wherein the composition is obtained by dissolving the active substance in a solvent, which is different from a solvent in which the solubility of the active substance shall be increased, in order to obtain a solution, mixing the solution with the crushed eggshell and optionally removing at least a part of the solvent, in which the active substance is dissolved.

The desired active substance is dissolved in a suitable solvent and the solution is applied to crushed eggshells. As pointed out above, the active substance can be soluble in one solvent but insoluble in another solvent. For preparing the composition, a solvent is chosen which dissolves the active substance because otherwise no sorbtion is possible. Such a suitable solvent furthermore can be pharmaceutically acceptable. Typical suitable solvents are ethanol or oils. The solvent can be evaporated. Stirring and/or heat and/or an extraction system for the solvent can be used to accelerate the process. After a nearly complete optional removal of the solvent, loaded eggshells are present as a powder, which can then be used directly or further processed into other formulations (suspensions, creams, gels, ointments, pastes, capsules, granules, pellets, tablets, drops, sprays, suppositories, lozenges, patches, pens, etc.).

By sorbing the active substance in or on the eggshells, the solubility of the active ingredient is increased, resulting in improved passive diffusion (bioavailability). The active substance can be released in a sustained manner, so that a long application is possible. Surprisingly, it was found that the addition of acid and glycerol to the composition boosts passive penetration.

In one embodiment, the removing of at least a part of the solvent is carried out by applying heat and/or reduced pressure. Generally, it is suitable but not necessary to completely remove the solvent. The solvent, in particular in small amounts, can be still present as long as it has no negative effect on the further use of the composition according to the present invention.

In one embodiment, the mixture of solvent and crushed eggshell is stirred. This stirring can be carried out before and/or during the solvent is removed. Stirring means to bring the components of a liquid into circular motion in order to mix them uniformly.

As pointed out above, the composition according to can be used for preparing suspensions, creams, gels, ointments, pastes, capsules, granules, pellets, tablets, drops, sprays, suppositories, lozenges, patches, pens.

The composition according to the present invention can be used for pharmaceuticals, healthcare, food, cosmetics, textile industry, paints and coatings, agriculture (for example for fertilizers), depending on the kind of the active substance.

The invention will be further illustrated by referring to the following examples and figures. It is pointed out that the examples and figures are intended for illustrating the invention but they shall not be construed to restrict the invention thereto.

Figure 1 shows fluorescence microscopy images of skin sections of untreated skin (A), treated with bulk material (B), physical mixture (C) and composition according to the present invention (D). 200× magnification, size scale corresponds to 50 µm.

Figure 2 shows the influence of additives on the penetration of curcumin. The addition of glycerol, as well as acid and glycerol significantly increased the penetration of curcumin into the skin (one-factor ANOVA with Bonferroni-Holm corrected post-hoc analysis).

### Example 1 Solubility

Curcumin is a poorly soluble substance and served as a surrogate for very poorly soluble active ingredients in this experiment. The aim was to demonstrate that the incorporation of poorly soluble substances into eggshells can improve their solubility. Curcumin was first incorporated into eggshell flour. Two different methods were used for this purpose. Method 1 - Curcumin was dissolved in ethanol and the solution was placed on eggshells. Ethanol was evaporated without stirring (HP). Method 2 - Curcumin was dissolved in ethanol and the solution was placed on the eggshells. Ethanol was evaporated with stirring (R). For comparison, curcumin was mixed with eggshell meal (physical mixture = PM) and added to water as a pure substance (bulk). From all samples, the amount of dissolved curcumin was measured. The results show that when curcumin was incorporated into the eggshells, the aqueous supernatants turned strongly yellow, i.e., curcumin was effectively dissolved in the water phase. No discolouration of the water phase occurred in the physical mixture and the pure substance - thus, no dissolution of curcumin occurred here. The dissolved amount of curcumin was determined at different time points by UV/Vis measurement. For method 1, the solubility for curcumin was about 100 times higher than the comparative solution with pure curcumin. For method 2, the solubility was approximately 50 times higher.

The loaded eggshells contained 10% (w/w) curcumin, i.e., 90% eggshell and 10% curcumin. The physical mixture contained similar amounts.

### Example 2 - biological effectiveness:

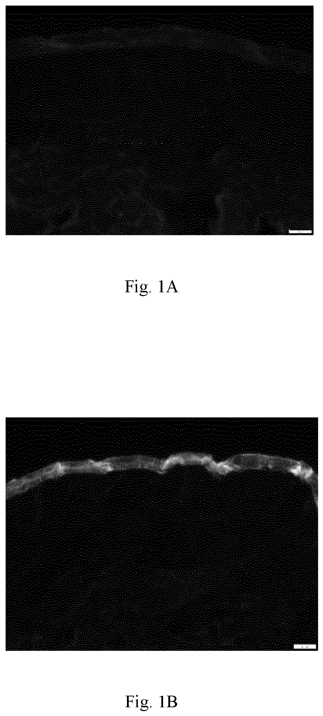

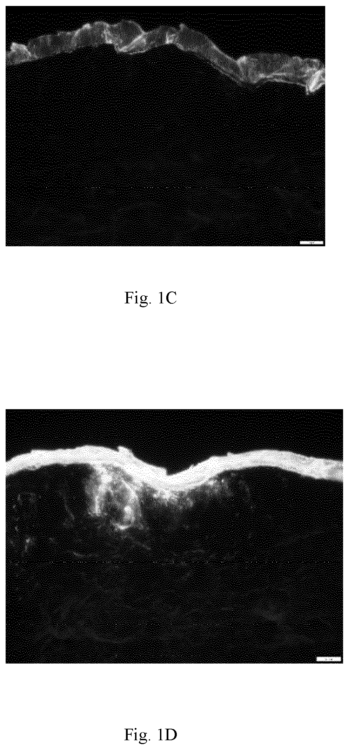

The aim was to show that passive penetration of poorly soluble active ingredients, i.e. their biological availability, can be improved by loading in eggshells. The dermal availability was tested in the ex-vivo pig ear model. Curcumin loaded eggshells (method 1 of example 1) were mixed with water in a 1:1 ratio and applied to fresh and uninjured pig skin. The amount of active ingredient penetrated was analysed after 4 h of penetration. Pure curcumin powder dispersed in water and the physical mixture served as comparison. Curcumin loaded in crushed eggshell could effectively penetrate into the skin and showed transdermal penetration. Pure curcumin powder and the physical mixture penetrated slightly into the upper layers of the skin and showed no transdermal penetration (Fig. 1A to 1D).

### Example 3 - optimized biological effectiveness through additives

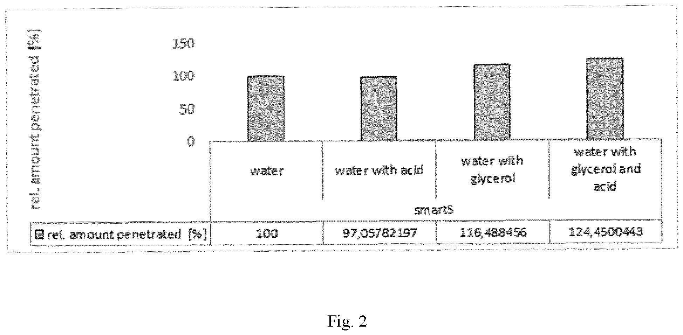

Surprisingly, it was found that the addition of glycerol and acid (citric acid) to the curcumin-loaded eggshell increased the biological availability of curcumin again by about 25% (Fig. 2) .

### Example 4 - Use for oral application:

Eggshell meal was manually compressed into tablets. It was shown that tablets of pharmaceutical quality - without the addition of further excipients - can be produced from eggshell meal by direct tableting.

## Claims

1. Composition for increasing the solubility of an active substance, comprising crushed eggshell, on or in which the active substance is sorbed.

2. The composition according to claim 1, wherein the composition is obtainable by dissolving the active substance in a solvent, which is different from a solvent in which the solubility of the active substance shall be increased, in order to obtain a solution, mixing the solution with the crushed eggshell and optionally removing at least a part of the solvent, in which the active substance is dissolved.

3. The composition according to claim 1 or 2, wherein the active substance is an active pharmaceutical ingredient.

4. The composition according to any of the preceding claims, wherein the crushed eggshell are from hen's eggs.

5. The composition according to any of the preceding claims, wherein the crushed eggshell is present as a ground, powdered, micronized or nanonized material.

6. The composition according to any of the preceding claims, wherein the crushed eggshell is present as a coarse-grained powder, a medium-fine powder, a fine powder, or a very fine powder.

7. The composition according to any one of the preceding claims, wherein the crushed eggshell is loaded with from 0.1% to 100% of the maximum loading capacity of the crushed eggshell with the active substance.

8. The composition according to any of the preceding claims, wherein the composition is present in suspensions, creams, gels, ointments, pastes, capsules, granules, pellets, tablets, drops, sprays, suppositories, lozenges, patches, pens.

9. The composition according to any of the preceding claims, further containing a compound selected from the group consisting of C3-C10 trihydroxy compound and/or an acid.

10. Method for preparing a composition for increasing the solubility of an active substance, comprising crushed eggshell, on or in which the active substance is sorbed, wherein the composition is obtained by dissolving the active substance in a solvent, which is different from a solvent in which the solubility of the active substance shall be increased, in order to obtain a solution, and mixing the solution with the crushed eggshell.

11. The method according to claim 10, wherein at least a part of the solvent is removed, in which the active substance is dissolved.

12. The method according to claim 11, wherein the removing of at least a part of the solvent is carried out by applying heat and/or reduced pressure.

13. The method according to any of claims 10 to 12, wherein the mixture of solvent and crushed eggshell is stirred.

14. Use of the composition according to any of claims 1 to 9 in suspensions, creams, gels, ointments, pastes, capsules, granules, pellets, tablets, drops, sprays, suppositories, lozenges, patches, pens.

15. Use of the composition according to any of claims 1 to 9 for pharmaceuticals, healthcare, food, cosmetics, textile industry, paints and coatings, agriculture.
